# EUROPEAN PATENT SPECIFICATION

(11) **EP 2 098 164 B1**
(45) Date of publication and mention of the grant of the patent: **22.04.2015**
(21) Application number: 09250570.0
(22) Date of filing: 27.02.2009
(51) Int. Cl.: A61B 5/04, A61B 5/0408

(54) **Biomedical electrode**
Biomedizinische Elektrode
Électrode biomédicale

(30) Priority: 06.03.2008 US 43266
(43) Date of publication of application: 09.09.2009
(73) Proprietor: Covidien LP, Mansfield, MA 02048 (US)
(72) Inventor: Garstka, Erick, Westfield, MA 01085 (US); Copp, Warren, Chicopee, MA 01013 (US); Dunagan, Jay, Wabasha, MN 55981 (US); Rowe, Chuck, Winona, MN 55987 (US)
(74) Representative: Gray, James

(56) References cited:
- WO-A-99/39635
- WO-A-2009/041496
- GB-A- 2 457 025
- US-A- 4 911 169

## Description

### BACKGROUND

### 1. Technical Field

The present disclosure generally relates to biomedical electrodes and, in particular, relates to a biomedical electrode capable of use in a garment.

### 2. Discussion of Related Art

Medical electrodes are used to transmit electrical signals between the body of a patient and external medical equipment, such as a monitoring, diagnostic, or stimulating device.

Biomedical electrodes are commonly used in therapeutic and diagnostic medical applications including, e.g., a variety of signal based rehabilitative procedures, electrocardiograph (ECG) or transcutaneous electrical nerve stimulation (TENS) procedures, maternal and/or fetal monitoring. Conventional biomedical electrodes are secured to the skin of a patient via a hydrogel and/or pressure sensitive adhesive. An electrical cable or lead wire is used to place the electrode in communication with an external electrical source. Various mechanisms for connecting a male/female terminal of the electrode to the complementary male/female terminal of the lead wire typically include "snap on" connectors, "pinch clip" arrangements, "twist on" couplings or magnetic couplings. A backside (i.e., a side opposite the hydrogel side) is typically provided with a non-conductive liner.

Accordingly, a need exists for an electrode including a first side supporting a hydrogel having a first adhesive quality or property for selective or removable fixation to the skin of a patient, and second side supporting a pressure sensitive adhesive (PSA) having a second adhesive quality or property for selective or removable fixation to a substrate, such as, for example, a garment or the like.

A further need exists for an electrode in which the adhesive properties of the PSA are greater than the adhesive properties of the hydrogel. Known electrodes are disclosed in WO 99/39635 and US4911169.

### SUMMARY

The invention is defined in independent claim 1 and dependent claims 2-10.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a schematic illustration of a garment for use in a medical, diagnostic, muscle stimulation, and/or therapeutic procedure, incorporating an electrode according to an embodiment of the present disclosure;
FIG. 2 is a cross-sectional, side elevational view of the electrode of FIG. 1;
FIG. 3 is a perspective view of the electrode of FIGS. 1 and 2, shown with layers separated;
FIG. 4 is a cross-sectional, side elevational view of an electrode; and
FIG. 5 is a perspective view of the electrode of FIG. 4, shown with layers separated.

### DETAILED DESCRIPTION OF EMBODIMENT

The exemplary embodiments of the electrodes as a component of a reusable multi-electrode garment system disclosed herein are discussed in terms of use in performing a surgical therapeutic or diagnostic procedure in delivering or collecting electrical signals relative to a subject. Such procedures are inclusive of, but, not limited to, a variety of signal based rehabilitative procedures, muscle stimulation, electrocardiograph procedures, maternal and/or fetal monitoring.

In the discussion that follows, the term "subject" refers to a human patient or other animal. The term "clinician" refers to a doctor, nurse or other care provider and may include support personnel.

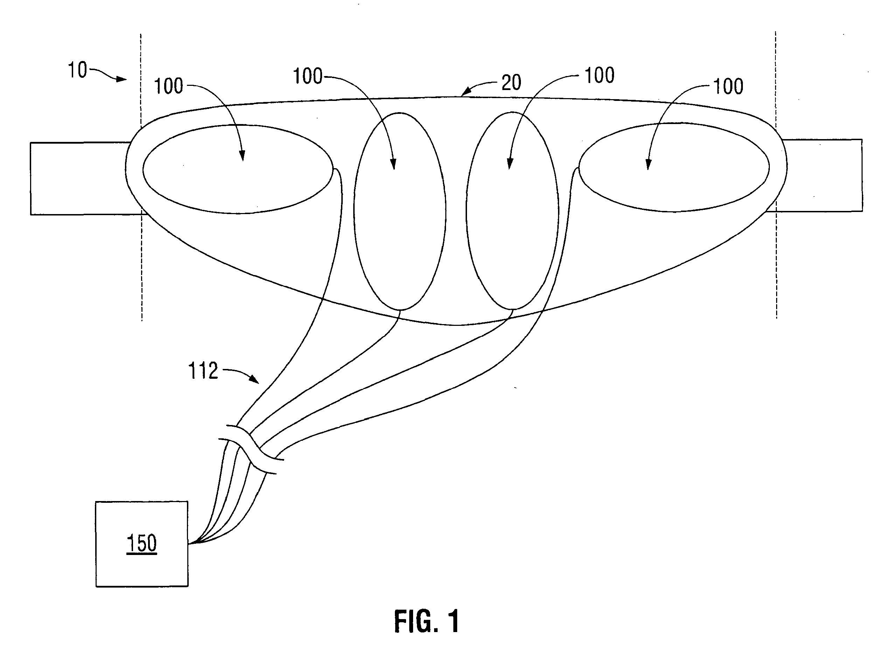

Referring now to the drawings wherein like components are designated by like reference numerals throughout the several views, FIG. 1 illustrates a garment 20 incorporating an electrode 100 in accordance with the principles of the present disclosure. Garment 20 may be any suitable garment member and may be in the form of a vest, shirt, gown, belt, wrapping or the like. Garment 20 may be worn during an electrotherapeutic, muscle stimulation, or medical monitoring procedure, and is subsequently removed after completion of the procedure. Garment 20 is intended to be reused for subsequent procedures. Garment 20 may be fabricated from cloth, polyester or the like and is non-conductive in nature. As illustrated in FIG. 1, in one embodiment, garment 20 may incorporate a plurality of electrodes 100 which may be secured to a surface of garment 20 (e.g., within the interior of the garment 20) at predefined positions relative to the subject to provide the desired therapeutic or diagnostic effect, either prior to the donning of garment 20 or after donning of garment 20.

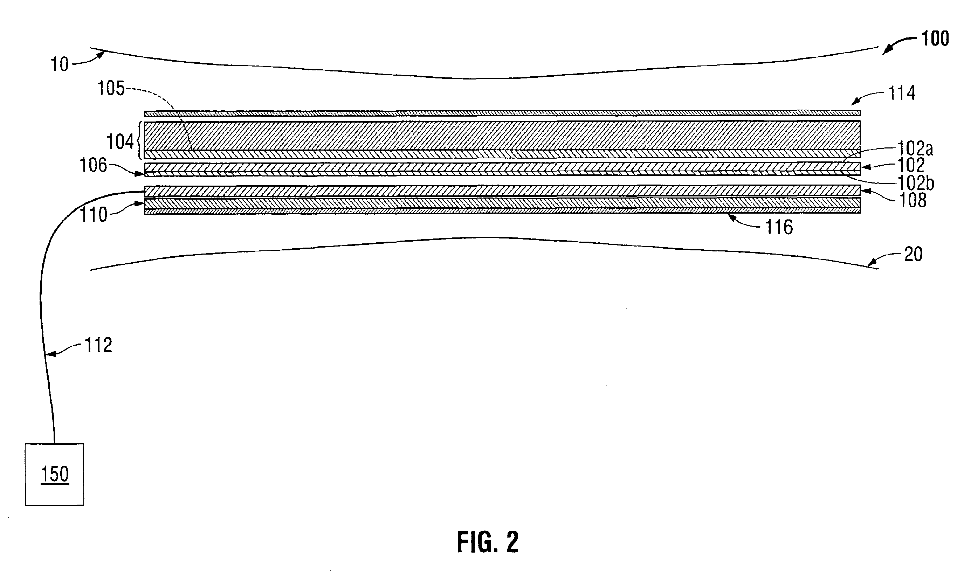

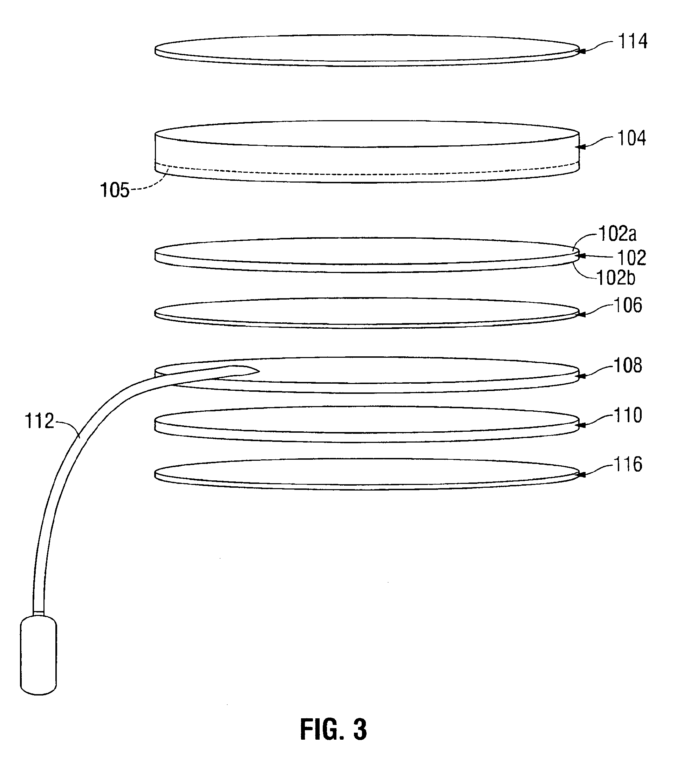

Referring now to FIGS. 2 and 3, one embodiment of the electrodes of the present disclosure will be discussed in detail. Leadwire style biomedical electrode 100 is configured for selective attachment or affixation to garment 20 and for selective attachment or affixation to a subject 10, e.g., to the skin of the subject 10. Electrode 100 includes a conductive member 102 defining a first or skin side 102a relative to the subject 10 and a second or garment side 102b, opposite first side 102a. Conductive member 102 may be made from a conductive carbon, aluminum, tin or any other suitable material. As a further alternative, conductive member 102 may comprise a conductive plastic material. Conductive member 102 may include a coating of a silver composition 106 on either or both the skin and garment sides 102a and 102b thereof, respectively (only silver composition 106 disposed on garment side 102b is shown).

Electrode 100 further includes a conductive composition 104 disposed adjacent skin side 102a of the conductive member 102 for application/adhesion to or contact with the skin of subject 10. Conductive composition 104 may be made from, for example, but not limited to, Promeon RG-63B hydrogel (TycoHealthcare Group LP d/b/a Covidien). As seen in FIGS. 2 and 3, in some embodiments, conductive composition 104 may incorporate a woven and/or nonwoven cloth or gauze material (e.g., scrim) 105 embedded therewithin or supporting the structure of the hydrogel. The conductive composition 104 may be any different commercially available conductive hydrogel.

In certain embodiments it may be desirable to provide a layer of adhesive (not shown) interposed between conductive member 102 and conductive composition 104.

A first or skin side release liner 114 is releasably secured to conductive composition 104. Release liner 114 can be made from a film or paper substrate having a release coating on one or both sides, such as, for example silicone. Release liner 114 protects and/or preserves conductive composition 104 (e.g., the hydrogel) and is removed prior to application on the skin of the subject 10. Release liner 114 may be applied to conductive composition 104 after use of electrode 100 to preserve the conductive composition 104 for subsequent use.

In an embodiment, electrode 100 may further include a reinforcement member 108 disposed adjacent garment side 102b of conductive member 102. In certain embodiments, reinforcement member 108 may overlie silver composition 106.

Electrode 100 further includes a layer of pressure sensitive adhesive (PSA) 110 disposed adjacent garment side 102b of conductive member 102. In an embodiment, pressure sensitive adhesive 110 overlies reinforcement member 108. Reinforcement member 108 may be in the form of a scrim comprising a non-conductive fabric material.

Pressure sensitive adhesive 110 (PSA) is adapted and or selected to enable selective attachment of electrode 100 to garment 20. The pressure sensitive adhesive 110 may be hot melt, acrylic, and/or rubber based adhesive. In one embodiment, pressure sensitive adhesive 110 is substantially a non-conductive, acrylic adhesive. A second release liner 116 is positioned to cover pressure sensitive adhesive 110. First and second release liners 114 and 116 may each be made from poly-coated paper having a thickness of about 0.127 millimeters (mm) (about 0.005 inches or 5 mils).

Electrode 100 further includes an electrical lead or lead wire 112, e.g., a lead wire having a pig tail configuration that is in electrical communication with at least conductive member 102 and to power supply or monitor 150 (see FIGS. 1 and 3). Electrical communication extends from lead wire 112 through the conductive member 102 (and silver composition 106) and through conductive composition 104 to subject 10.

As described above, electrode 100 may be selectively attached to a garment 20 as a component of a reusable multi-electrode garment system. Specifically, a plurality of electrodes 100 may be selectively adhered to an interior of garment 20 at predetermined locations depending on the medical therapeutic or diagnostic procedure being performed on the subject 10. In one embodiment, the adhesive characteristics or properties of pressure sensitive adhesive 110 relative to garment 20 is greater than the adhesive characteristics or properties of conductive composition 104 (e.g., the hydrogel) relative to the skin. Since the bond between conductive composition 104 and conductive member 102 is more aggressive than the bond between conductive composition 104 and patient skin, removal of garment 20 from the skin of subject 10 will cause removal of electrodes 100 from the skin of subject 10 prior to removal of electrodes 100 from garment 20. As such, garment 20 and electrodes 100 may be removed simultaneously.

That is, conductive composition 104 possesses an adhesive strength permitting repeated removal of garment 20 and electrode 100 from the subject 10 without substantially interfering with the bond between pressure sensitive adhesive 110 and garment 20. With this arrangement, garment 20 and associated electrodes 100 may be reused in subsequent procedures during a course of therapy or diagnostic applications.

Referring to FIG. 2, in one method of application, release liner 116, adjacent garment side 102b of conductive member 102, is removed from the pressure sensitive adhesive 110 and the respective electrode 100 is applied to garment 20 at a predefined location. Such predefined locations will correspond to a selected area of the body of the subject to be treated or monitored. A number of electrodes 100 may be mounted to garment 20 at various preselected locations. Thereafter, garment 20 is applied to the subject 10 with the electrodes 100 being positioned at the desired body locations. Release liner 114 adjacent conductive composition 104 may be removed either before or after garment 20 is donned. Pressure may be applied to the external surface of the garment 20 adjacent each electrode 100 to secure conductive composition 104 to the skin of subject 10. A therapeutic or diagnostic treatment may then be performed. Upon completion of the procedure, garment 20 is removed. Due to the relatively greater bond existing between pressure sensitive adhesive 110 and conductive member 102, as compared to between conductive composition 104 and conductive member 102, electrodes 100 are peeled away from the skin of subject 10 upon removal of garment 20. Release liner 114 may then be reapplied to the conductive composition 104 to preserve the conductive composition for a subsequent use.

Another method of application or use may include the steps of removing release liner 114 disposed on conductive composition 104 of each electrode 100 to be used, placing or affixing each electrode 100 on the skin of subject 10 at the desired location, removing release liner 116 disposed on pressure sensitive adhesive 110 of each electrode 100, placing garment 20 on subject 10, and applying pressure to garment 20 to affix garment 20 to pressure sensitive adhesive 110 of each electrode 100. Following placement or fixation of garment 20 to electrodes 100, electrical energy may be transmitted to electrodes 100, as is commonly known, to perform electrotherapy, stimulation and the like. Following the transmission of the electrical energy to electrodes 100, garment 20 may be removed from subject 10. As garment 20 is removed from the patient, electrodes 100 are peeled away from or removed from the skin of subject 10. In particular, due to the higher adhesive properties of pressure sensitive adhesive 110 as compared to conductive composition 104, electrodes 100 will more readily peel away from the skin of subject 10 as compared to garment 20. In other words, the aggressiveness of the adhesion of electrode 100 to garment 20, via pressure sensitive adhesive 110, is greater than the aggressiveness of the adhesion of electrode 100 to the skin of subject 10, via conductive composition 104.

Following removal of garment 20, original release liners 114 or replacement release liners (not shown) may be applied to conductive composition 104 to thereby protect conductive composition 104. The release liners 114 may remain in place until the next course of treatment for the patient. In this manner, garment 20 is now provided with electrodes 100 which are properly located (or located at the same position) on the individual subject 10 for each subsequent course of treatment.

In the event that one electrode 100 is defective, said defective electrode 100 may be removed from garment 20 and replaced with a new electrode 100. In this manner, the entire garment does not have to be replaced.

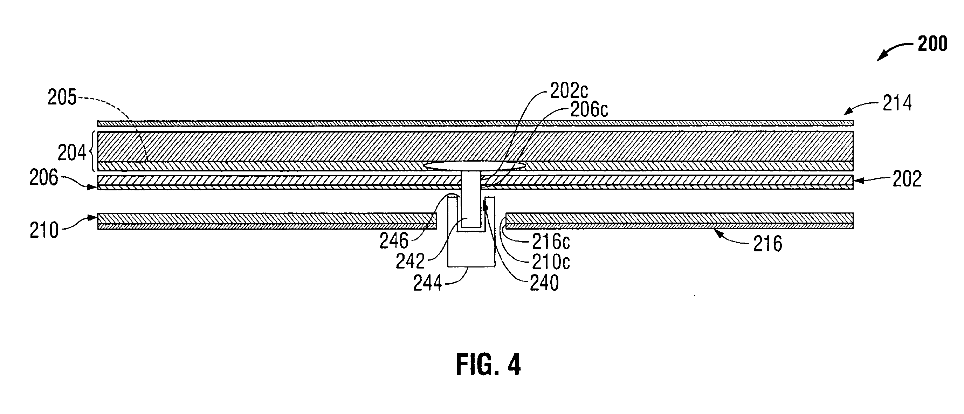

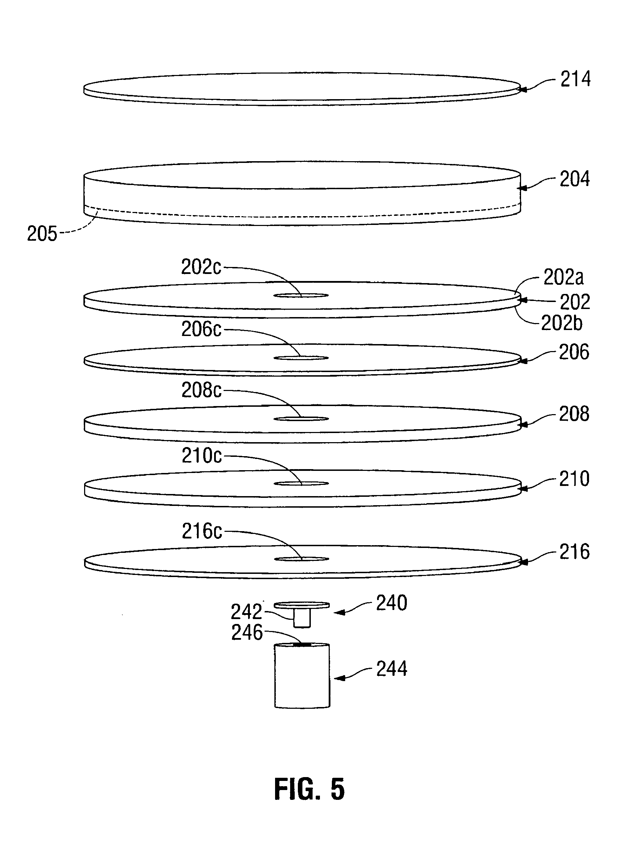

Turning now to FIGS. 4 and 5, there is illustrated an electrode 200. More particularly, electrode 200 is configured for attachment to garment 20 and to subject 10 and includes a conductive member 202 defining first side 202a and second side 202b. Conductive member 202 has at least one aperture 202c formed therein that is in communication with a second side 20a of garment 20. A conductive composition 204 is disposed on a first or skin side of electrode 200. A connector component 240 is disposed against conductive composition 204 and may, in certain embodiments (as seen in FIG. 4), be embedded within conductive composition 204. Connector component 240 may be conductive and may define a male terminal or male pin 242 as shown.

The electrode may further include a coating of silver provided on at least one of the first and second sides of the conductive member. In certain embodiments, electrode 200 may include a layer of silver composition 206 overlying second side 202b of conductive member 202, on a side opposite conductive composition 204.

As seen in FIG. 4, electrode 200 may be provided without a reinforcement member, or as seen in FIG. 5, electrode 200 may be provided with a reinforcement member 208 overlying silver composition 206. As seen in FIG. 4, a non-conductive pressure sensitive adhesive 210 may be disposed on silver composition 206 of electrode 200, or as seen in FIG. 5, non-conductive pressure sensitive adhesive 210 may be disposed on reinforcement member 208.

Pressure sensitive adhesive 210 secures electrode 200 relative to the garment 20 in a similar manner to that described in the electrode embodiment of FIGS. 2 and 3 and permits repeated removal and reuse without substantially affecting the bond created between pressure sensitive adhesive 210 and garment 20 (see FIG. 2). As can be appreciated from the foregoing description, a plurality of electrodes 200 may be attached to the interior of garment 20 at desired locations depending on the procedure contemplated.

As seen in FIGS. 4 and 5, conductive composition 204 may incorporate a woven and/or nonwoven cloth or gauze material (e.g., scrim) 205 embedded therewithin or supporting the structure of the conductive composition (e.g., hydrogel).

Each of silver composition 206 and pressure sensitive adhesive 210, and optionally reinforcement member 208, may be provided with a respective aperture 206c, 210c, and 208c that is in general alignment with aperture 202c of conductive member 202 for accommodating pin 242.

A second connector component 244 may be at least partially positioned within aperture 210c of pressure sensitive adhesive 210 and mechanically couples with connector component 240. In an alternate embodiment, if connector component 240 is non-conductive, an electrical interface may be established via second connector component 244 and conductive member 202. Second connector component 244 is thus in electrical contact with conductive member 202 via first connector component 240. Second connector component 244 is also electrically conductive. In one embodiment, second connector component 244 includes a female receptacle 246 which receives male terminal or pin 242 of first connector component 240 in a snap-type or threaded relation therewith to couple components 240, 244 with one another. Conductive member 202 may be interposed between first and second connector components 240, 244. Other arrangements for coupling first and second connector components 240, 244 are also envisioned including "pinch clip" arrangements, "twist on" couplings or the like. It is also envisioned that second connector component 244 may include the male pin or terminal and first connector component 240 may include the associated female receptacle to receive the male terminal to couple the components.

First and second connector components 240 and 244 may be used to electrically connect electrode 200 to a terminal of garment 20. First and second connector components 240, 244 may be in the form of a suitable snap style connector adapted to mechanically and electrically couple with a complementary female terminal (not shown) of garment 20.

Electrode 200 may be provided with first and second release liners 214, 216 to cover conductive composition 204 and pressure sensitive adhesive 210, respectively, in a similar manner to release liners 114, 116 discussed hereinabove.

The invention also provides an electrode system for selective use with a subject, the electrode system comprising:
a garment having a first side and a second side; and
at least one electrode for selective attachment to the first side of the garment and to the subject, the electrode including:
   a conductive member defining a first side and a second side;
   a conductive composition disposed on the first side of the conductive member, wherein the conductive composition has a first adhesive strength; and
   a pressure sensitive adhesive disposed on the second side of the conductive member, wherein the pressure sensitive adhesive has a second adhesive strength that is greater than the adhesive strength of the conductive composition;
   wherein the electrode is interposable between the garment and the subject such that the conductive composition is adherable to the subject and the pressure sensitive adhesive is adherable to the garment, and whereby, following use of the system, upon removal of the garment from the subject the electrode is removed only from the subject.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure.

## Claims

1. An electrode system (10) for selective use with a subject, the electrode system comprising:
a garment (20) having a first side and a second side; and
a plurality of replaceable electrodes (100) for selective attachment to the garment (20) and a subject (10), each electrode comprising:
a conductive member (102) defining a first side (102a) and a second side;
a conductive composition (104) disposed on the first side of the conductive member, wherein the conductive composition has a first adhesive strength;
a pressure sensitive adhesive (110) having a second adhesive strength that is greater than the adhesive strength of the conductive composition;
wherein each of the plurality of electrodes are interposed between the first side of the garment and the subject such that the conductive composition is adhered to the subject and the pressure sensitive adhesive is adhered to the garment;
an electrical lead for electrical communication with the conductive member (102);
the system further includes
a reinforcement member (108) arranged on the second side (102b) of conductive member (102) and having the pressure sensitive adhesive (110) disposed thereon, the reinforcement member (108) being a scrim of a non-conductive material.

2. An electrode system according to claim 1, further comprising a release liner (114) selectively removably adhered to a surface of the conductive composition (104).

3. An electrode system according to any one of the preceding claims, wherein the pressure sensitive adhesive (110) is one of conductive and non-conductive.

4. An electrode system according to any one of the preceding claims, wherein at least one of the pressure sensitive adhesive (110) and the conductive composition (104) is a hydrogel.

5. An electrode system according to any one of the preceding claims, further comprising a reinforcement member (180) supporting the conductive composition (104).

6. An electrode system according to any one of the preceding claims, further comprising a coating of silver provided on at least one of the first and second sides (102a, 102b) of the conductive member (102).

7. An electrode system according to any preceding claim wherein the electrical lead is a pig-tail style electrical lead.

8. An electrode system according to any preceding claim wherein the electrical lead is in electrical communication with the conductive composition (104).

9. An electrode system according to any preceding claim wherein the electrical lead (112) is a snap-style electrical lead having a first end in electrical communication with the conductive composition (104).

10. An electrode system according to claim 9, further comprising a scrim (105, 205) supporting the conductive composition (104, 204).

## Patentansprüche

1. Elektrodensystem (10) zur selektiven Verwendung bei einem Subjekt, wobei das Elektrodensystem umfasst:
ein Kleidungsstück (20) mit einer ersten Seite und einer zweiten Seite; und
eine Mehrzahl von auswechselbaren Elektroden (100) zur selektiven Befestigung am Kleidungsstück (20) und einem Subjekt (10), wobei jede Elektrode umfasst:
ein leitendes Element (102), das eine erste Seite (102a) und eine zweite Seite definiert; und
eine leitende Zusammensetzung (104), die auf der ersten Seite des leitenden Elements angeordnet ist, wobei die leitende Zusammensetzung eine erste Haftfestigkeit aufweist;
einen Haftklebstoff (110) mit einer zweiten Haftfestigkeit, die größer als die Haftfestigkeit der leitenden Zusammensetzung ist;
wobei jede der Mehrzahl von Elektroden derart zwischen die erste Seite des Kleidungsstücks und das Subjekt eingefügt wird, dass die leitende Zusammensetzung an das Subjekt geklebt wird und der Haftklebstoff an das Kleidungsstück geklebt wird;
eine elektrische Leitung zur elektrischen Kommunikation mit dem leitenden Element (102);
wobei das System ferner umfasst:
ein Verstärkungselement (108), das auf der zweiten Seite (102b) des leitenden Elements (102) angeordnet ist und den Haftklebstoff (110) darauf angeordnet aufweist, wobei das Verstärkungselement (108) ein Gitterstoff eines nichtleitenden Materials ist.

2. Elektrodensystem nach Anspruch 1, ferner umfassend eine Trennschicht (114), die selektiv entfernbar an eine Oberfläche der leitenden Zusammensetzung (104) geklebt ist.

3. Elektrodensystem nach einem der vorhergehenden Ansprüche, wobei der Haftklebstoff (110) eines von leitend oder nichtleitend ist.

4. Elektrodensystem nach einem der vorhergehenden Ansprüche, wobei mindestens eines von dem Haftklebstoff (110) und der leitenden Zusammensetzung (104) ein Hydrogel ist.

5. Elektrodensystem nach einem der vorhergehenden Ansprüche, ferner umfassend ein Verstärkungselement (180), das die leitende Zusammensetzung (104) trägt.

6. Elektrodensystem nach einem der vorhergehenden Ansprüche, ferner umfassend eine Silberbeschichtung, die auf mindestens einer der ersten und zweiten Seiten (102a, 102b) des leitenden Elements (102) vorgesehen ist.

7. Elektrodensystem nach einem der vorhergehenden Ansprüche, wobei die elektrische Leitung eine elektrische Pigtail-Leitung ist.

8. Elektrodensystem nach einem der vorhergehenden Ansprüche, wobei die elektrische Leitung in elektrischer Kommunikation mit der leitenden Zusammensetzung (104) ist.

9. Elektrodensystem nach einem der vorhergehenden Ansprüche, wobei die elektrische Leitung (112) eine elektrische Schnappleitung mit einem ersten Ende in elektrischer Kommunikation mit der leitenden Zusammensetzung (104) ist.

10. Elektrodensystem nach Anspruch 9, ferner umfassend einen Gitterstoff (105, 205), der die leitende Zusammensetzung (104, 204) trägt.

## Revendications

1. Système d'électrode (10) destiné à être utilisé sélectivement avec un patient, le système d'électrode comprenant :
un vêtement (20) ayant un premier côté et un second côté ; et
une pluralité d'électrodes remplaçables (100) pour la fixation sélective au vêtement (20) et à un patient (10), chaque électrode comprenant :
un élément conducteur (102) définissant un premier côté (102a) et un second côté ;
une composition conductrice (104) disposée sur le premier côté de l'élément conducteur, dans lequel la composition conductrice a une première résistance d'adhésion ;
un adhésif sensible à la pression (110) ayant une seconde résistance d'adhésion qui est supérieure à la résistance d'adhésion de la composition conductrice ;
dans lequel chacune de la pluralité d'électrodes est intercalée entre le premier côté du vêtement et le patient de sorte que la composition conductrice adhère au patient et que l'adhésif sensible à la pression adhère au vêtement;
un fil électrique pour la communication électrique avec l'élément conducteur (102) ;
le système comprend en outre :
un élément de renforcement (108) agencé sur le second côté (102b) d'élément conducteur (102) et ayant l'adhésif sensible à la pression (110) disposé sur ce dernier, l'élément de renforcement (108) étant un canevas léger d'un matériau non conducteur.

2. Système d'électrode selon la revendication 1, comprenant en outre un revêtement antiadhésif (114) fixé de manière sélectivement amovible à une surface de la composition conductrice (104).

3. Système d'électrode selon l'une quelconque des revendications précédentes, dans lequel l'adhésif sensible à la pression (110) est l'un parmi un adhésif conducteur et un adhésif non conducteur.

4. Système d'électrode selon l'une quelconque des revendications précédentes, dans lequel au moins l'un parmi l'adhésif sensible à la pression (110) et la composition conductrice (104) est un hydrogel.

5. Système d'électrode selon l'une quelconque des revendications précédentes, comprenant en outre un élément de renforcement (180) supportant la composition conductrice (104).

6. Système d'électrode selon l'une quelconque des revendications précédentes, comprenant en outre un revêtement d'argent prévu sur au moins l'un des premier et second côtés (102a, 102b) de l'élément conducteur (102).

7. Système d'électrode selon l'une quelconque des revendications précédentes, dans lequel le fil électrique est un fil électrique en tire-bouchon.

8. Système d'électrode selon l'une quelconque des revendications précédentes, dans lequel le fil électrique est en communication électrique avec la composition conductrice (104).

9. Système d'électrode selon l'une quelconque des revendications précédentes, dans lequel le fil électrique (112) est un fil électrique de type à encliquetage ayant une première extrémité en communication électrique avec la composition conductrice (104).

10. Système d'électrode selon la revendication 9, comprenant en outre un canevas léger (105, 205) supportant la composition conductrice (104, 204).
